(19) Europäisches Patentamt
European Patent Office
Office européen des brevets

(11) **EP 4 684 758 A2**

(12) **EUROPEAN PATENT APPLICATION**

(43) Date of publication:
**28.01.2026 Bulletin 2026/05**

(21) Application number: **25213501.7**

(22) Date of filing: **19.02.2020**

(51) International Patent Classification (IPC):
***A61F 2/00*** (2006.01)

(52) Cooperative Patent Classification (CPC):
**A61F 2/72; A61B 5/397; A61F 2/582; G06F 3/015;
G06F 3/016; G06F 3/017; G16H 40/40;
G16H 40/63;** A61B 2505/09; A61F 2002/704;
A61F 2002/7615; G06N 7/02; G06N 20/00

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.02.2019 US 201962807306 P**

(62) Document number(s) of the earlier application(s) in
accordance with Art. 76 EPC:
**20758760.1 / 3 927 282**

(71) Applicant: **Coapt LLC
Chicago, IL 60654 (US)**

(72) Inventors:
• **LOCK, Blair Andrew
Chicago (US)**
• **CUMMINS, Frank Daniel II
Chicago (US)**
• **HARGROVE, Levi John
Chicago (US)**
• **THOMPSON, John Arthur IV
Madison (US)**

(74) Representative: **Murgitroyd & Company
165-169 Scotland Street
Glasgow G5 8PL (GB)**

Remarks:
This application was filed on 04.11.2025 as a
divisional application to the application mentioned
under INID code 62.

(54) **ELECTROMYOGRAPHIC CONTROL SYSTEMS AND METHODS FOR THE COACHING OF EXOPROSTHETIC USERS**

(57) Systems and methods are described for the coaching of users through successful calibration of a myoelectric prosthetic controller. The systems and methods are comprised of, and/or utilize, hardware and software components to input and analyze electromyography (EMG) based signals in association with movements, and to calibrate and output feedback about the signals. The hardware is further comprised of an apparatus for the detection of EMG signals, a prosthesis, an indicator, and a user interface. The software is further comprised of a user interface, a pattern recognition component, a calibration procedure, and a feedback mechanism. The systems and methods facilitate calibration of a myoelectric controller and provides the user with feedback about the calibration including information of the signal inputs and outputs, and messages about connected hardware and how to optimize signal data.

**EP 4 684 758 A2**

**Description**

## RELATED APPLICATION

This application claims the benefit of U.S. Provisional Application No. 62/807,306, as filed on February 19th, 2019.

## FIELD OF THE DISCLOSURE

The present invention (e.g., the electromyographic control system) is generally related to the field of electromyography (EMG) and the coaching of exoprostheses users, and more specifically the coaching of these users through the usage of electromyograph control systems.

## BACKGROUND

When initially setting up a control system for a prosthesis, it is necessary to calibrate the control based on the EMG signals produced by the user. Also, during normal daily use of a myoelectric exoprosthesis, control of the prosthesis may degrade. This degradation can occur from many different sources such as muscle exhaustion, humidity, or the prosthesis socket shifting. Because of these frequently changing variables, a myoelectric prosthesis user requires a way to regain accurate control of their device. Traditionally, when a myoelectric prosthesis user needs to have their system initially set up or recalibrated, it involves going to a prosthetist and having an in person appointment, which may take many hours to make sure that the system is correctly responding to their EMG signals. This is not always a possibility and very inconvenient for the user.

For the foregoing reasons, there is a need for electromyograph control systems and methods for the coaching of exoprosthetic users.

## BRIEF SUMMARY

A need for the invention of the present disclosure arises from the necessity of having a method to rapidly coach a user for successful calibration of a myoelectric prosthetic controller. The embodiments described herein describe electromyograph control systems and methods that allow a myoelectric prosthetic user to quickly recalibrate his or her myoelectric prosthetic controller on the fly, without the need to go to a specialist. In addition, the electromyograph control systems and methods described herein allow a user to receive feedback on his or her calibration, which allows the user to have the most accurate control of their device. The electromyograph control systems and methods of the present disclosure can be utilized for these, and other embodiments as described herein, as such electromyograph control systems and methods coaches the user to quickly and accurately (and initially) calibrate and recalibrate a myoelectric prosthetic controller and/or myoelectric prosthetic control system. In various embodiments described herein, during a given calibration session, the electromyograph control systems and methods guide a user through the process to calibrate the EMG signals produced from their muscle movements for use with a myoelectric prosthetic control system and may then provide feedback on the information gathered from the calibration session. Calibrating the EMG signals produced from the user's muscle movements include any one or more of changing, altering, adding, removing, or augmenting a particular movement to improve the quality of signals received by the myoelectric prosthetic control system. Calibrating the EMG signals produced from the user's muscle movements may also include the prosthetic control system using pattern recognition capabilities to recognize characteristics within the user's EMG signals to determine when the user is making a particular motion. In various embodiments, based on the quality of the information received from the calibration session, the user may receive feedback telling the user that he or she has successfully calibrated all aspects, or, additionally or alternatively, to adjust certain parameters and recalibrate. In some embodiments, the entire procedure of calibration using the disclosed electromyograph control systems and methods may take less than 5 minutes, and can be performed many times a day, making it more advantageous for prostheses users.

The representative embodiments of the present invention provide numerous advantages over the commonly used methods of calibrating myoelectric prosthetic control systems. The general purpose of the invention is to provide a method for simple and quick recalibration and training of a myoelectric prosthetic control system. In various embodiments, the many novel features described result in a new method for rapidly coaching the user through a successful calibration procedure for the user's myoelectric control system.

To attain this, the present invention generally comprises a system for the coaching of a user through the set up and calibration of a myoelectric prosthetic control system. In some embodiments, the disclosed system may further be comprised of a system for the input of signal data from a plurality of electrodes. In still further embodiments, the disclosed system may also be further comprised of a button that includes a housing, an indicator, and a tactile interface. In addition, the disclosed system may also further be comprised of a software component that includes a user interface and a pattern recognition component.

In various embodiments, the disclosed system may collect signal data from the input of a plurality of electrodes. These electrodes may be made from an electrically conductive material. The plurality of electrodes may be coupled to the software component in a way that enables communication of the signal data.

In still further embodiments, an indicator on the button that may comprise part of the system may send out an indication to the user. This indication can be displayed as a visual stimulus, auditory stimulus, and/or a tactile stimulus. The tactile interface of the button allows the user to initiate the calibration procedure, for example, when a component is manually depressed by a user to initiate the calibration procedure or when an accelerometer is activated by the user to initiate the calibration procedure.

In various embodiments, the software component of the system may include a user interface (UI) that is used for providing feedback of information to the user. This feedback of information may be comprised of a quality metric that is used in identifying an objective level of calibration quality, and/or a message that identifies one or more probable causes of poor signal data. The message may also contain data used as an indication of the cause for non-optimal signal data input, and a recommended procedure for optimizing the signal data input. The user interface may also contain information for a calibration procedure, and a set of instructions to guide the user through the calibration procedure. The user can also utilize the user interface to monitor the signal data, in real time. The user interface allows the user to select various combinations of prostheses, select various movements supported by the prostheses, and provides indications of signal data input and output. Along with allowing the user to select various prostheses and movements, the user interface provides the user with the identification of the connected hardware, and the status of the connected hardware. In some embodiments, the user Interface is displayed as a virtual application. This application can be contained on a smartphone or computer and is installed using content from the internet or uploadable content from a physical disk or drive. The installation methods have compatibility with most digital operating systems. The software component also comprises a method to initiate the calibration procedure from the virtual application.

In various embodiments, a component of the software for the system may include a pattern recognition component. The pattern recognition component may be used to receive, analyze, and output the signal data. In some embodiments, the pattern recognition component also contains an adaptive machine learning system to recognize the users unique signal data. This adaptive machine learning system recognizes a user's unique signal data and references it to a particular motion performed by the user. It gives an identification to the signal data and motion and categorizes this information. In some embodiments, the pattern recognition component may be coupled to the user interface for communication of the categorized signal data to the user. In some embodiments, the adaptive machine learning system categorizes sections of user experience data to determine if particular motions require more calibration events compared to others. In embodiments where the machine learning system or component may prompt or initiate new solutions or require new motions for or by the user to increase the calibration quality of a particular motion.

In accordance with the above, and with the disclosure herein, the present disclosure includes improvements in computer functionality or in improvements to other technologies at least because the present disclosure describes that, e.g., prosthetic devices, and their related various components, may be improved or enhanced with the disclosed electromyograph control systems and methods that provide calibration and more accurate control of the prosthetic devices for their respective users. That is, the present disclosure describes improvements in the functioning of a prosthetic device itself or "any other technology or technical field" (e.g., the field of electromyography) because the disclosed electromyograph control systems and methods improve and enhance operation, and reduce error rate, of prosthetic devices by introducing user recalibration procedures for correcting non-optimal EMG signal data to eliminate errors and malfunctions typically experienced over time by prosthetic devices lacking such systems and methods. This improves over the prior art at least because such previous systems were error-prone as they lack the ability for rapidly coaching the user through a successful calibration procedure for the user's myoelectric control system.

In addition, the present disclosure includes applying various features and functionality, as described herein, with, or by use of, a particular machine, e.g., a myoelectric prosthetic controller, a prosthetic device, a button, and/or other hardware components as described herein.

Moreover, the present disclosure includes effecting a transformation or reduction of a particular article to a different state or thing, e.g., transforming or reducing the calibration and/or operation of a prosthetic device from a non-optimal or error state to an optimal or calibrated state.

Still further, the present disclosure includes specific features other than what is well-understood, routine, conventional activity in the field, or adding unconventional steps that demonstrate, in various embodiments, particular useful applications, e.g., electromyograph control systems and methods for the coaching of exoprosthetic users.

Advantages will become more apparent to those of ordinary skill in the art from the following description of the preferred embodiments which have been shown and described by way of illustration. As will be realized, the present embodiments may be capable of other and different embodiments, and their details are capable of modification in various respects. Accordingly, the drawings and description are to be regarded as illustrative in nature and not as restrictive.

## BRIEF DESCRIPTION OF THE DRAWINGS

The objects, features and advantages of the present invention and additional embodiments will be more readily appreciated upon reference to the following disclosure when considered in conjunction with the accompanying drawings, wherein like reference numerals are used to identify identical components in the various views.

The Figures described below depict various aspects of the system and methods disclosed therein. It should be understood that each Figure depicts an embodiment of a particular aspect of the disclosed system and methods, and that each of the Figures is intended to accord with a possible embodiment thereof. Further, wherever possible, the following description refers to the reference numerals included in the following Figures, in which features depicted in multiple Figures are designated with consistent reference numerals.

There are shown in the drawings arrangements which are presently discussed, it being understood, however, that the present embodiments are not limited to the precise arrangements and instrumentalities shown, wherein:

Figure 1 illustrates an example flowchart representing use and operation of an electromyograph control system to coach a user through a calibration procedure and feedback of electromyography signals, in accordance with various embodiments disclosed herein.

Figure 2 illustrates an example representation of a virtual user interface demonstrating electrode connectivity, calibration quality, and virtual arm and/or prosthetic mode switching, in accordance with various embodiments disclosed herein.

Figure 3 illustrates an example representation of a virtual user interface demonstrating each connected device, calibration strength for multiple motions, and system of Figure 1 for the coaching of exoprosthetic users, including messages and training tips, in accordance with various embodiments disclosed herein.

Figure 4A illustrates an example representation of the user of Figure 1 with a prosthetic wrist and hand with electrodes, and two separate modalities for accessing the virtual user interface(s) of either Figures 2 and/or 3, and in accordance with various embodiments disclosed herein.

Figure 4B illustrates a further example representation of the user of Figure 4A with a prosthetic wrist and hand with electrodes as attached to the user, in accordance with various embodiments disclosed herein.

Figure 5A illustrates an example representation of signal data received from calibration by the user and analyzed by the system of Figure 1 for the coaching of exoprosthetic users, in accordance with various embodiments disclosed herein.

Figure 5B illustrates a further example representation of the signal data of Figure 5A including a categorization of the signal data according to indicated motions as shown by Figure 5A.

Figure 6A illustrates a representation of an electronic button, including housing, indicator, and tactile interface, of the system of Figures 1 and 4A in accordance with various embodiments disclosed herein.

Figure 6B illustrates an embodiment of the button of Figure 6A shown together with the system of Figure 1 and 4A in accordance with various embodiments disclosed herein.

The Figures depict preferred embodiments for purposes of illustration only. Alternative embodiments of the systems and methods illustrated herein may be employed without departing from the principles of the invention described herein.

## DETAILED DESCRIPTION

While the present invention is susceptible of embodiment in many different forms, there are shown in the drawings and will be described herein in detail specific exemplary embodiments thereof, with the understanding that the present disclosure is to be considered as an exemplification of the principles of the invention and is not intended to limit the invention to the specific embodiments illustrated. In this respect, before explaining at least one embodiment consistent with the present invention in detail, it is to be understood that the invention is not limited in its application to the details of construction and to the arrangements of components set forth above and below, illustrated in the drawings, or as described in the examples. Methods and apparatuses consistent with the present invention are capable of other embodiments and of being practiced and carried out in various ways. Also, it is to be understood that the phraseology and terminology employed herein, as well as the abstract included below, are for the purposes of description and should not be regarded as limiting.

As disclosed in various embodiments herein, a system 100 is described for the coaching of exoprosthetic users. System 100 may also be referred to herein as "the system" and/or "the electromyograph control system." System 100 is generally comprised of hardware and software components to input and analyze EMG based signals in association with movements of a user (e.g., user 123), and to calibrate and output feedback about the signals. System 100, and portions thereof, including its various hardware and software components, are illustrated herein by the provided Figures.

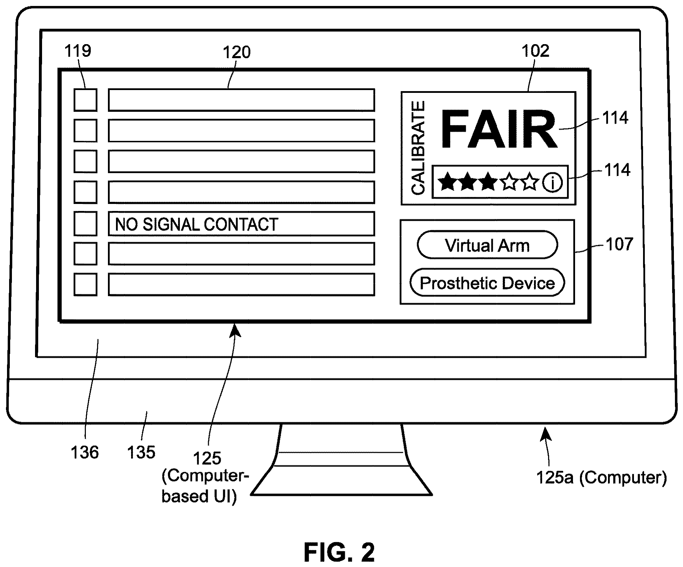

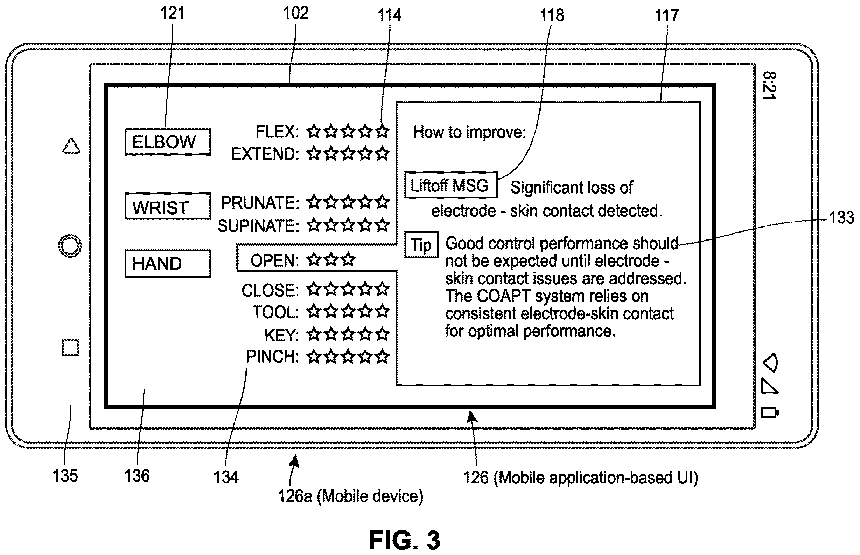

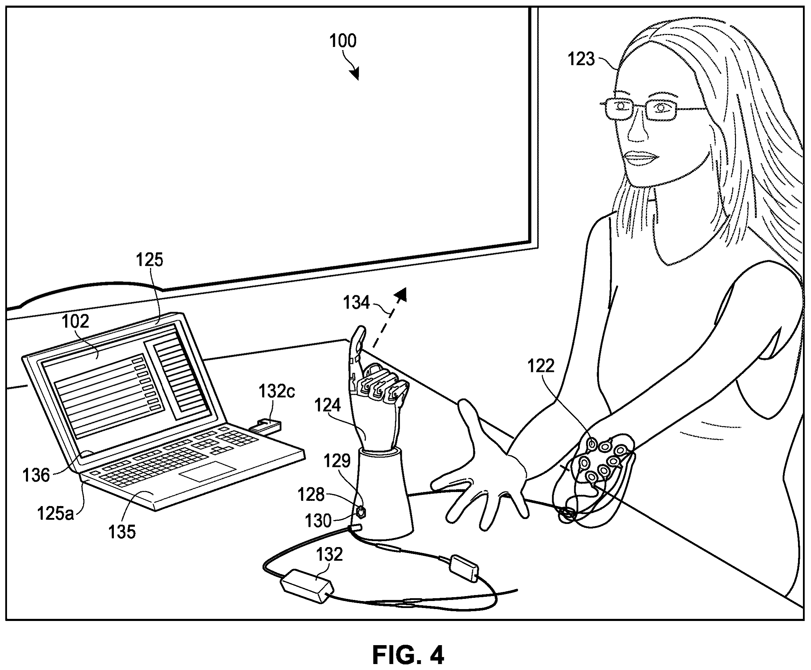

For example, Figure 4A illustrates an example representation of a user (e.g., user 123) with a prosthetic wrist and hand (e.g., prosthetic device 124) with electrodes 122, and two separate modalities (e.g., computer 125a of Figure 2 and mobile device 126a of Figure 3 (not shown)) for accessing the virtual user interface(s) 125 and 126 of either Figures 2 and/or 3, and in accordance with various embodiments disclosed herein.

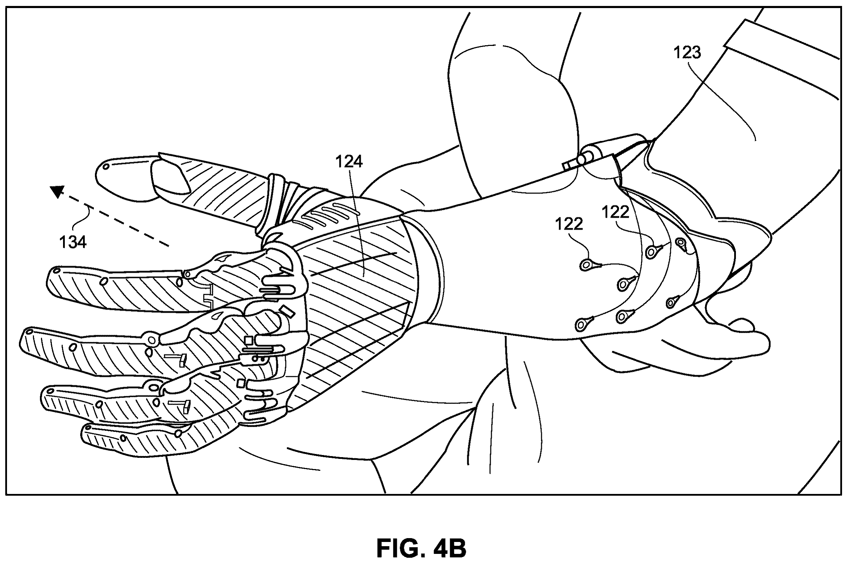

In addition, Figure 4B illustrates a further example representation of the user of Figure 4A with a prosthetic wrist and hand (e.g., prosthetic device 124) with electrodes 122 as attached to user 123, and in accordance with various embodiments disclosed herein.

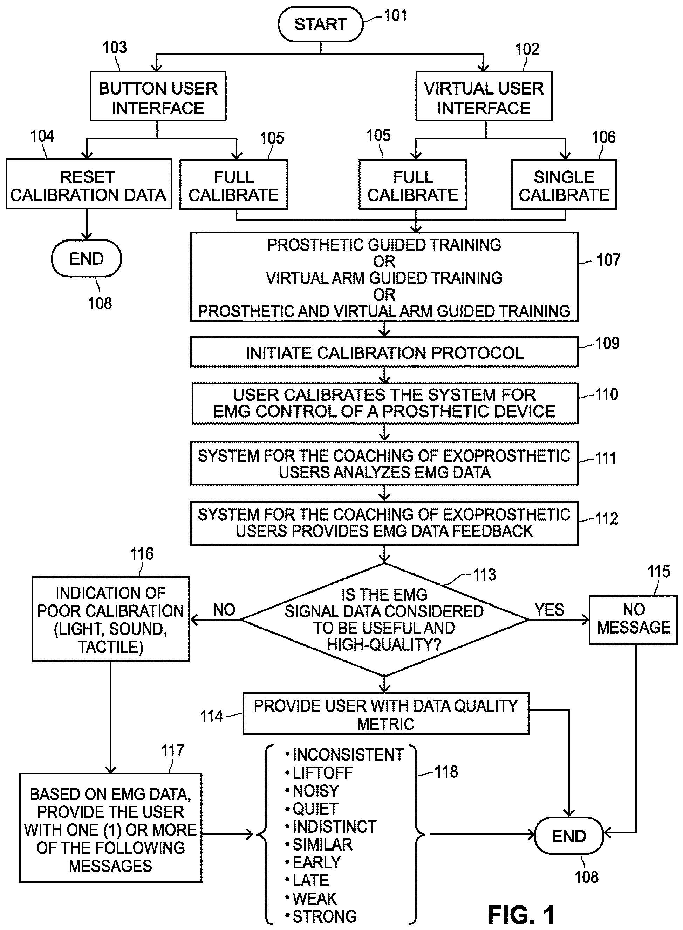

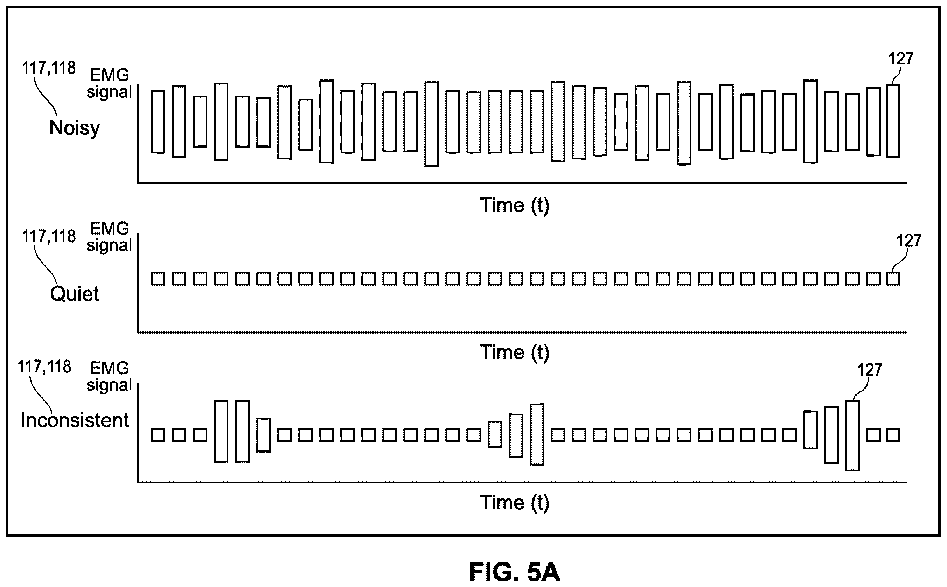

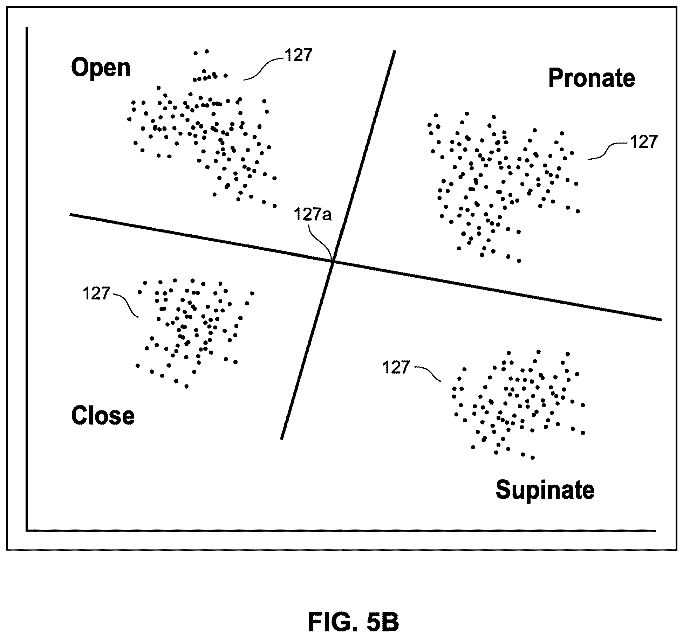

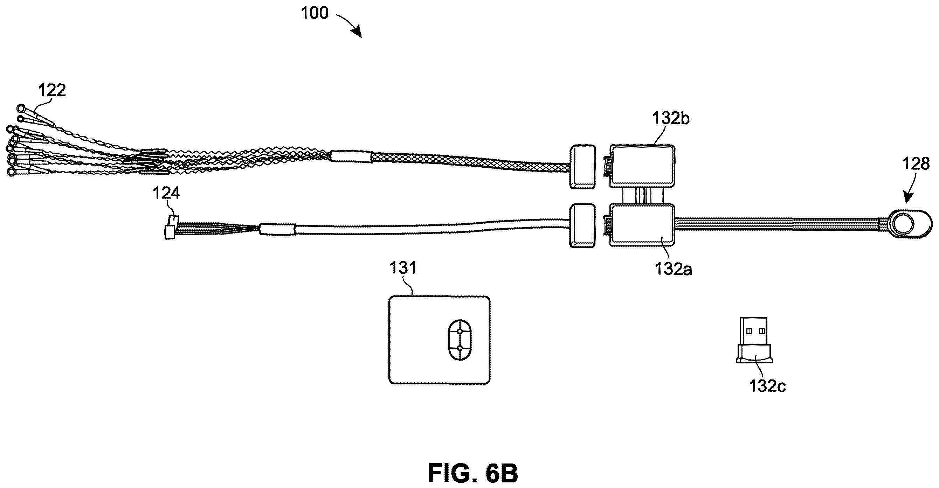

As illustrated by at least Figures 4A, 4B, and 6B, system 100 comprises a plurality of electrodes 122, a button 128, and a software component 136 that are coupled to a prosthetic device 124. For example, system 100 may be communicatively coupled to prosthetic device 124 via wired or wireless hardware and/or software components (e.g., via connection module 132, mobile application-based UI 126, mobile device 126a, etc.). For example, as shown in Figure 6B, connection module 132 may include hardware component 132c represented as wireless communication component 132c, such as a USB based wireless transmitter communicating via BLUETOOTH, WIFI, or other radio-based communication standard. The prosthetic device 124 is coupled to system 100, wherein signal data 127, for example as shown herein for Figures 5A and 5B, may be collected from plurality of electrodes 122. In various embodiments, electrodes 122 may be configured as EMG electrodes. For example, as shown for Figures 1 (118) and Figure 5A (117, 118), signal data 127 may indicate, define, or classify signal data (e.g., EMG signal data of movements of a user over time) as any one or more of "inconsistent," "liftoff," "noisy," "quiet," "indistinct," "similar," "early," "late," "weak," or "strong," etc. For example, Figure 5A illustrates an example representation of signal data 127 received from calibration by user 123 and analyzed by system 100 for the coaching of exoprosthetic users. The plurality of electrodes 122 may collect signal data 127 and transfer it to software component 136.

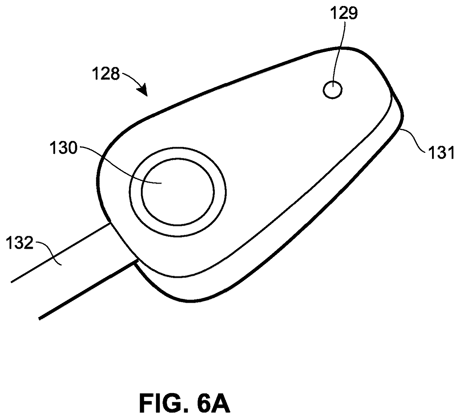

Figure 1 illustrates an example flowchart representing use and operation of system 100 to coach a user through a calibration procedure and feedback of electromyography signals. As illustrated for Figure 1, button 128 may be implemented as button user interface 103. In addition, as shown for Figure 6A, button 128 may comprise an indicator 129 (e.g., an LED or visual indicator), where user 123 can be notified as to the quality of the signal data 127 through an auditory, tactile, or visual stimulus. Button 128 may be coupled to prosthetic device 124 for interaction by user 123.

Figure 6B illustrates an embodiment of the button of Figure 6A shown together with system 100, and various components, including electrodes 122, connector to prosthetic device 124, button 128, button housing 131, and connection module 132 (e.g., where the embodiment of Figure 6B is illustrated as comprised of various hardware components 132a, 132b, and 132c). In various embodiments, a myoelectric prosthetic controller, which may be configured or calibrated to control prosthetic device 124, may be included in hardware components 132a and/or 132b. In some embodiments, button 128 may comprise a tactile interface 130 wherein user 123 can interact with system 100 and control the calibration of the prosthetic device 124 (110) as described herein for Figure 1. For example, Figure 1 illustrates user calibration 110 of system 100 where a user (e.g., user 123) calibrates system 100 for EMG control of a prosthetic device (e.g., prosthetic device 124). Tactile interface 130 is coupled to input/output connection module 132 through which information can be communicated between the button 128 and the software component 136. In some embodiments, the components of the button 128 may be enclosed within a housing 131 (e.g., a button housing) of substantially rigid material.

Software component 136 may be comprised of a user interface (e.g., computer-based user-interface 125 as illustrated by Figure 2, and/or mobile application-based user-interface 126 as illustrated by Figure 3), and an analyzing (111) pattern recognition algorithm as illustrated by and described for Figures 1-3, 5A, and 5B. In various embodiments, a user interface (e.g., computer-based UI 125 and/or mobile application-based UI 126) is accessed through starting (101) the process for usage of a prosthetic device 124. As shown for Figure 1 illustrates the initiation (101) of use of the electromyographic control system (system 100) for the coaching of exoprosthetic users. For example, in various embodiments, first, a user 123 must decide whether he or she is going to initiate calibration (e.g., via reset calibration procedure (104) or calibration protocol (109)) through a button user interface (103) or a virtual user interface (102).

Once the method of interface has been selected, user 123 must decide whether to reset their calibration data (104) of prosthetic device 124, fully calibrate (105) the device (e.g., prosthetic device 124), or calibrate a single portion (106) of the device (e.g., prosthetic device 124). As shown by Figure 1, user 123 may initiate a reset of the user's calibration data (104) via button user interface 103. In some embodiments, a reset of the user's calibration data (104) via button user interface 103 may delineate an end (108) of a session or use of the electromyograph control system (system 100). Additionally or alternatively, user 123 may initiate full calibration (105) of the user's signal data via either button user interface (103) or virtual user interface (102). Still further, additionally or alternatively, user 123 may initiate calibration of a single motion (106) of the user's signal data via virtual user interface (102). To guide user 123, user 123 may then decide whether to be guided (107) by the prosthetic device 124 (e.g., an exoprosthetic device), the virtual user interface 125,126, or both the prosthetic device 124 and the virtual user interface 125, 126. For example, as shown for Figure 2, the user 123 may select a method for guidance (107) for guiding the user through exoprosthetic calibration via a prosthetic arm guided training, virtual arm guided training, or both prosthetic and virtual arm guided training.

In addition, Figure 2 illustrates an example representation of a virtual user interface 102 demonstrating electrode connectivity (e.g., via user interface indicator(s) 119 that an electrode 122 is connected to system 100), calibration quality (e.g., via data quality metrics 114), and virtual arm and/or prosthetic mode switching (e.g., via guidance 107). In some embodiments, virtual user interface 102 may include user interface indicator(s) 119 may indicate whether or not a certain of electrodes 122 is connected to system 100. As shown for Figure 2, if an electrode is not connected, then a virtual user interface 102 may display a user interface indicator 120 regarding the status of a connected electrode 122 (e.g., "no signal contact").

Once the user 123 has selected the method for guidance 107, the electromyograph control system (system 100) will begin or initiate a calibration protocol 109. User 123 may then go through a select series of motions, i.e., calibration classes, such as, e.g., an indicated motion 134, including any one or more of an elbow motion ("flex" and/or "extend"), wrist motion ("pronate" and/or "supinate"), and/or a hand motion ("open," "close," "tool," "key," and/or "pinch") as illustrated by Figure 3, and as prompted by the calibration protocol 109 through the selected method of guidance 107, which generates signal data 127, and calibrates system 100. For example, in various embodiments, system 100 (e.g., by software component 136 and/or by pattern recognition component) may analyze (111) signal data 127 (e.g., EMG data) in relation to the prosthetic device 124 (e.g., an exoprosthetic device) and each prompted motion (e.g., elbow motion ("flex" and/or "extend"), wrist motion ("pronate" and/or "supinate"), and/or hand motion ("open," "close," "tool," "key," and/or "pinch") as illustrated by Figure 3) from the calibration protocol 109. System 100 may then categorize signal data 127 and prepare it for user 123 as meaningful feedback (112) on the calibration quality. In this way, system 100 provides signal data feedback (112).

Referring to Figure 1, signal data may be analyzed (111) by system 100 (e.g., by software component 136 and/or by pattern recognition component) to determine its use and quality. If signal data 127 is determined by system 100 (e.g., by software component 136 and/or by pattern recognition component) to be adequate (113) (e.g., useful and high quality as illustrated by any of Figures 1, 2, 3, 5A and/or 5B) for prosthetic 124 use, then no message (115) may be provided to the user 123, and the calibration for the exoprosthetic system may be ended (108), which may delineate an end of current use or session of the electromyograph control system (system 100). That is, system 100 (e.g., by software component 136 and/or by pattern recognition component) determines the signal data is adequate and provided the user with no message (115). In some embodiments, as illustrated by Figure 2, system 100 may provide the user with a data quality metric 114 (e.g., a "fair" quality metric and/or three star rating) to indicate adequate signal data 127. As a further example, as illustrated by Figure 3, system 100 may provide the user with a data quality metric 114 (e.g., any of the high (e.g., five) star ratings for any of indicated motions 134, e.g., "flex," "extend," "pronate," "supinate," "close," "tool," "key," "pinch," etc.) to indicate adequate signal data 127.

If, however, signal data 127 is determined (e.g., by software component 136 and/or analyzing pattern recognition algorithm) to be inadequate (116) (e.g., a poor calibration determined as illustrated by any of Figures 1, 2, 3, 5A and/or 5B) then the user 123 may be provided with an indication of poor calibration (116) through either the button user interface 103 or the virtual user interface 102. That is system 100 (e.g., by software component 136 and/or by pattern recognition component) may determine that signal data 127 is inadequate (116) and may provide the user with an indication or message of poor calibration. For example, as shown for Figure 1, system 100, via button user interface 103/button 128, may provide a light, sound, or tactic feedback to the user indicating inadequate/poor calibration. As a further example, as shown for Figure 3, system 100, via virtual user interface 102, provides the user with a data quality metric 114 (e.g., a three star quality metric for signal data 127 collected for an "open" indicated motion), which, in some cases, may indicate that signal data 127 captured was inadequate (116).

In various embodiments, signal data 127 may be determined by system 100 (e.g., by software component 136 and/or by pattern recognition component) as inadequate because it is defined or classified as "noisy," "quiet," or "inconsistent," etc., e.g., over a given time period, as illustrated for Figure 5A (117, 118) and Figure 1 (118). For example, any one or more of inadequate or non-optimal signal data 127 (e.g., "noisy," "quiet," or "inconsistent," etc., as shown for Figure 5A or Figure 1 (118)) may result from poor electrode-skin contact (or other contact) of electrodes 122 to user 123 causing inadequate or poor signal quality, and, therefore inadequate signal data 127 and inadequate calibration. For example, as indicated by Figure 3, significant loss of electrode-skin contact may be detected by system 100, where system 100 may rely on consistent electrode-skin contact for optimal performance.

In either case (i.e., for an adequate calibration or an inadequate calibration), as illustrated by any of Figures 1, 2, and/or 3, the user 123 is provided with a quality metric 114 based on the collected signal data 127. For example, Figure 5B illustrates an example representation of signal data 127 of Figure 5A including a categorization of the EMG signal data according to example indicated motions 134 of Figure 5A. For example, as illustrated for Figure 5B, software component 136 of system 100 may include a pattern recognition component. The pattern recognition component may be used to receive, analyze, and output signal data 127. In some embodiments, the pattern recognition component may also contain an adaptive machine learning system or model to recognize the users unique signal data 127. The adaptive machine learning system may recognize a user's (e.g., user 123) unique signal data 127 and reference it to a particular motion (e.g., a calibration class) performed by the user (e.g., any of "open," pronate," "close," and/or "supinate" as shown for Figures 5A and 5B). The pattern recognition component may give an identification to the signal data 127, and related motion (e.g., indicated motion 134), and categorize or group (127a) this information, e.g., across unique signal data 127 values unique to a given user, as represented by Figure 5B. The pattern recognition component may be coupled to a user interface (e.g., 125 or 126) for communication of the categorized signal data 127, via one or more messages, to user 123.

In various embodiments, pattern recognition component (or, alternatively, and more generally, the electromyographic software component as described herein) is configured to determine statistics from EMG signal data during calibration for providing recommendations as described herein. For example, based on statistical analysis of the EMG signal data (e.g., signal data 127), common user errors (e.g., "liftoff") can be identified, and solutions suggested. Statistics determined and analyzed by pattern recognition component may include covariance of time domain features, signal magnitude, variation of signal magnitude over time, separation index between motion classes, frequency of electrode liftoff, and a variety of others. Additionally, or alternatively, fuzzy logic may be applied to these statistics to indicate when a specific, but yet widely observed error, is likely to be occurring, e.g., as caused by user error and/or user contact with electrodes 122. In such embodiments, fuzzy logic may be applied to each statistic. Additionally, or alternatively, statistics generated from EMG signal data may be converted into a value compatible with fuzzy logic by assigning a stochastic value indicating a confidence that the statistic is outside an expected range. These values (e.g., as described for Figure 5B) may be analyzed by pattern recognition component for specific combinations that indicate common problems (e.g., "liftoff"). For example, such problems may include issues with signal distinctness, issues demonstrating muscle contractions during the recording, over-exertion, and poor electrode connection, timing issues during calibration, noise issues, and signal similarity issues, e.g., as described and shown herein for Figures 1 (118), Figure 5A (117, 118), and/or Tables 1-6. Each issue may correspond to a calibration class, which together, or individually, be assigned a rating (e.g., data quality metrics 114) to be displayed to the user as described. Generally, pattern recognition component analyzes the severity of each common issue, and the confidence that the given issue is the root cause of poor control, in order to select which message types or otherwise messages to send to the user. As described herein, such messages include a rating (e.g., data quality metrics 114) indicating expected performance, as well as directed messages (e.g., messages 117) indicating what was detected and what can be done to correct for the mistakes in subsequent calibrations.

Analysis of EMG signal data 127 of a user by pattern recognition component (or, alternatively, and more generally, the electromyographic software component as described herein) may further be described with respect to the following Tables 1-5, which describe the signal data collection, derived features (statistics), fuzzy logic scaling, fuzzy logic implementation, and rating and issue selection.

Data Collection

The pattern recognition component (or, alternatively, and more generally, the electromyographic software component as described herein), e.g., implementing the calibration quality algorithm, needs input EMG signal data 127 from the calibration session task to analyze. Such data may be used to build a classifier for the pattern recognition component, while others may be collected for implementation of the calibration quality algorithm. Additionally, or alternatively, for single class calibrations (e.g., only one class is calibrated), some data will only be needed from the relevant class, while others will be needed from all classes regardless. Table 1, below, provides examples of data or data types (e.g., EMG signal data 127) that may be collected, determined, or otherwise received as described herein. It is to be understood that the data or datatypes in the below table or provided by way of example only, and are not limiting. Additional or different data, sizes, etc. may be utilized that is consistent with the disclosure herein.

**TABLE 1 (Data Collection)**

| Item | Data | Description | Size | Single Class Requirement |
|------|------|-------------|------|--------------------------|
| 1 | All-Calibration Covariances for each Class | One covariance matrix for each class in the classifier, as used to build the classifier | Kx(fxc) x(f x c) x 4B = K x 12544B | May be required for all Classes |

(continued)

| Item | Data | Description | Size | Single Class Requirement |
|---|---|---|---|---|
| 2 | All-Calibration Centroids for each Class | One centroid vector for each class in the classifier, as used to build the classifier | K x (f x c) x 4B = K x 224B | May be required for all Classes |
| 3 | All-Calibration Number of Frames for each Class | One value for each class in the classifier indicating the number of frames used to calculate the above data | K x 4B | May be required for all Classes |
| 4 | This-Calibration Centroids for each Class | One centroid vector for each class in the most recent calibration only representing the data collected during this calibration | k x (f x c) x 4B = k x 244B | This class |
| 5 | This-Calibration No-Motion Mean Relative Value (MRV) Variance | One vector representing the variance of the MRV data collected during no-motion recording of this calibration; Typically for full calibration only | c x 4B = 32B | Typically required for Full Calibration only |
| 6 | Reallocation Data for each recording | A set of values for each recording in the most recent calibration indicating the proportion of recorded frames reallocated during each part of a partition of the recording | k x r x p x 4B = k x 24B | This Class |

In the above Table 1, K is total number of classes recognized by the classifier (e.g., typically K = 2 to 20), k is the number of classes used for calibration (e.g., typically 1 or K), f is the number of measured data features per channel (e.g., 7 standard), c is the number of channels (e.g., 8 channels), which may be associated with electrodes 122 and/or EMG signal data 127 received via electrodes 122, and B is the number of Bytes (in a memory, as described herein), where each value may be either a float or uint32 (e.g., each 4 Bytes). As shown in the "size" column above, the pattern recognition component (or, alternatively, and more generally, the electromyographic software component as described herein), e.g., implementing the calibration quality algorithm, requires minimum amounts of memory (e.g., a few bytes of information in some cases), allowing for the pattern recognition component (or, alternatively, and more generally, the electromyographic software component as described herein) to be implemented on devices having minimum memory and/or processing resources. Each of this data may be collected during a calibration session ad described herein.

Derived Features (Statistics)

With respect to an additional portion of calibration quality algorithm, pattern recognition component (or, alternatively, and more generally, the electromyographic software component as described herein), may process the EMG signal data 127 to generate derived data features, e.g., including statistics as used by fuzzy logic implementations as described herein.

**TABLE 2 (Derived Features (Statistics))**

| Item | Derived Features | Description | Size | Utilized Data (Items of Table 1) |
|---|---|---|---|---|
| 7 | Separation Index | For each pair of one class in the most recent calibration and one class in the classifier (excluding pairs of identical classes), the Mahalanobis distance between their centroids (Items 4 and 2 of Table 1 respectively) using the average of their covariances (Item 1) | k x (K - 1) x 4B | 1,2,4 |
| 8 | Separation Index Scaling Factor | For each Separation Index, a scaling factor that scales inversely to the number of frames of data contributing to the averaged covariances (Item3) | k x (K - 1) x 4B | 3 |
| 9 | MRV Ratio | Ratio of the average MRV of each motion class to the average MRV of the no motion class. | k_m x 4B | 2 |

(continued)

| Item | Derived Features | Description | Size | Utilized Data (Items of Table 1) |
|---|---|---|---|---|
| 10 | No-Motion Variability | Average across channels of the ratio of the square root of MRV variance to the MRV mean in the no-motion class | 4B | 4,5 |

In the above Table 2, and similarly with respect to Table 1, K is total number of classes recognized by the classifier (e.g., typically K = 2 to 20), k is the number of classes used for calibration (e.g., typically 1 or K), k_m is the number of motion classes (e.g., classes for detecting motion, e.g., ("open," "close," "tool," "key," and/or "pinch") as illustrated by Figure 3 in the calibration (typically 0m, 1, or K-1), and B is the number of Bytes (in a memory, as described herein), where each value may be either a float or uint32 (e.g., each 4 Bytes).

As described for Table 2 (Item 7), a separation index may be computed by pattern recognition component (or, alternatively, and more generally, the electromyographic software component as described herein) with the following formula:

$$\sqrt{(Class1Mean - Class2Mean)^T * CombinedCovariance^{-1} * (Class1Mean - Class2Mean)}$$

Fuzzy Logic Conversion

With respect to an additional portion of calibration quality algorithm, pattern recognition component (or, alternatively, and more generally, the electromyographic software component as described herein), may covert EMG signal data 127, including any one or more of EMG signal data 127, including data of Table I, and/or feature data of Table II. The conversion prepares the data for fuzzy logic implementation(s) as further described herein. Generally, fuzzy logic may rely on all variables being between 0 and 1, inclusive, heuristically representing the likelihood, confidence, or severity with which a given feature applies. In some cases, two polar opposite features may be represented with one variable between -1 and 1, where 0 represents that neither feature applies. The conversion may be implemented by a piecewise linear function, including, for example, the following function:

$$f(x) = 0 \; if \; x < a$$

or

$$1 \; if \; x > b$$

or

$$\frac{(x - a)}{b - a} if \; a < x < b$$

In the above function, $a$ is a lower bound and $b$ is an upper bound. Table 3 below represents fuzzy features that may be used by the calibration quality algorithm of pattern recognition component:

**TABLE 3 (Fuzzy Logic Conversion)**

| Item | Fuzzy Features | Description | Bounds | Size | Utilized Data (Items of Table 2) |
|---|---|---|---|---|---|
| 11 | Fuzzy Separation Index | Fuzzy conversion of the Separation Index, with bounds modified by the Separation Index Scaling Factor | 1 for values less than s*2.125 0 for values greater than s*3.4 | k x (K-1) x 4B | 7,8 |
| 12 | Fuzzy MRV Ratio | Fuzzy conversion of the MRV Ratio | 1 for values greater than 8 -1 for values less than 1.2 0 for values between 2 and 4.5 | k_m x 4B | 9 |
| 13 | Fuzzy No-Motion Variability | Fuzzy conversion of the No-Motion Variability | 1 for values greater than 0.3 -1 for values less than 0.09 0 for values between 0.14 and 0.2 | 4B | 10 |
| 14 | Fuzzy Early Reallocation | The average of the difference between the percentage of frames reallocated early and the period where fewest frames were reallocated | N/A | k_m x 4B | 6 |
| 15 | Fuzzy Late Reallocation | The average of the difference between the percentage of frames reallocated late and the period where fewest frames were reallocated | N/A | k_m x 4B | 6 |
| 16 | Fuzzy Total Reallocation | The percentage of frames reallocated | N/A | - x 4B | 6 |

In the above Table 3, and similarly with respect to Tables 1 and 2, K is total number of classes recognized by the classifier (e.g., typically K = 2 to 20), k is the number of classes used for calibration (e.g., typically 1 or K), k_m is the number of motion classes (e.g., classes for detecting motion, e.g., ("open," "close," "tool," "key," and/or "pinch") as illustrated by Figure 3 in the calibration (typically 0m, 1, or K-1), s is the Separation Index Scaling Factor (Item 8 of Table 2), and B is the number of Bytes (in a memory, as described herein), where each value may be either a float or uint32 (e.g., each 4 Bytes).

Fuzzy Logic Implementation

With respect to an additional portion of calibration quality algorithm, pattern recognition component (or, alternatively, and more generally, the electromyographic software component as described herein), may determine common issues (e.g., "too weak") that are prevalent in the data signal by implementing fuzzy logic operations on the fuzzy features of Table 3. The pattern recognition component (or, alternatively, and more generally, the electromyographic software component as described herein) may implement the following fuzzy logic operations across the fuzzy features of Table 3:

$$NOT(X) = 1 - X \qquad (1)$$

$$AND(X, Y) = X * Y \qquad (2)$$

$$OR(X, Y) = X + Y - X * Y \qquad (3)$$

Implementation of these fuzzy logic operations yield the following fuzzy feature results and related conclusions (for the given indicated operation). These fuzzy feature results and related conclusions generally correspond to classifications of Figures 1 (118) and Figure 5A (117, 118) as described herein.

**TABLE 4 (Fuzzy Logic Implementation)**

| Item | Fuzzy Feature | Conclusion | Operations | Class Type |
|------|---------------|------------|------------|------------|
| 17 | Quiet No-Motion | No-motion recording appears too relaxed | 13(negative only) | No-Motion |
| 18 | Noisy No-Motion | No-Motion recording appears to include motion | AND(13(positive only), 16) | No-Motion |
| 19 | Low Signal | The signal is too similar to no signal | OR(11(this/no-motion), 12(negative only)); may not be a final operation | Motion |
| 20 | Too Weak | The signal is too weak | AND(16, 19) | Motion |
| 21 | Inconsistent | The signal cuts out | AND(16, NOT(19)) | Motion |
| 22 | Indistinct | Many motion classes are similar | Mean(11(this/motions)) | Motion |
| 23 | Too Strong | Contractions are too intense | AND(12(positive only), 22) | Motion |
| 24 | Nearest Neighbor | A motion is too similar | Max(11(this/motions)) | Motion |
| 25 | Started Late | Beginning of recording is reallocated | 14 | Motion |
| 26 | Ended Early | End of recording is reallocated | 15 | Motion |

Rating and Issue Selection

With respect to an additional portion of calibration quality algorithm, pattern recognition component (or, alternatively, and more generally, the electromyographic software component as described herein), may select what gets displayed. For each class, a signal will be sent from pattern recognition component (or, alternatively, and more generally, the electromyographic software component as described herein) to software component 136, which may render or display the result, as described herein, on a user interface (e.g., computer-based user-interface 125 as illustrated by Figure 2, and/or mobile application-based user-interface 126 as illustrated by Figure 3), a rating (from value or rating 1 to 5) (e.g., data quality metrics 114)) and/or one or more messages.

While each issue may be identified via a fuzzy logic implementation, as described above, in some embodiments, some issues or classes may be determined or set as more severe than others. In such embodiments, pattern recognition component may multiply an issue or class by a scaling factor to determine its final priority and maximum rating penalty. Examples of scaling factors and penalty scalings are illustrated in Table 5 below. The fuzzy features and items of Table 5 correspond to those of Table 4, where the scaling factors and/or penalty scalings are applied to the output values for the fuzzy features of Table 4. It is to be understood that additional and/or different such scaling factors and penalty scalings may be used.

**TABLE 5 (Rating and Issue Selection)**

| Item | Fuzzy Feature | Priority Scaling | Penalty Scaling | Class Type |
|------|---------------|------------------|-----------------|------------|
| 17 | Quiet No-Motion | 0.5 | 2 | No-Motion |
| 18 | Noisy No-Motion | 1 | 4 | No-Motion |
| 19 | Too Weak | 1.2 | 4 | Motion |
| 20 | Inconsistent | 1.2 | 4 | Motion |
| 21 | Indistinct | 1 | 3 | Motion |
| 22 | Too Strong | 1 | 2 | Motion |
| 23 | Nearest Neighbor | 1 | 3 | Motion |
| 24 | Started Late | 1 | 2 | Motion |
| 25 | Ended Early | 1 | 2 | Motion |
| 26 | Quiet No-Motion | 0.5 | 2 | No-Motion |

Generally, each message (e.g., as described for Table 6 herein and Figures 1 (118) and Figure 5A (117, and 118) has its own rating and issue threshold. If the values of the fuzzy features (as determined with or without priority and/or penalty scaling) as described above for Table 5 and 6 cross a threshold of a rating and issue threshold of a message, then the message is displayed by software component 136, as described herein, which may render or display the result (a rating (from value or rating 1 to 5) (e.g., data quality metrics 114)) and/or one or more messages) on a user interface (e.g., computer-based user-interface 125 as illustrated by Figure 2, and/or mobile application-based user-interface 126 as illustrated by Figure 3).

Each of the fuzzy features of Tables 4 and 5 generally correspond to classifications of Figures 1 (118) and Figure 5A (117, 118) as described herein, such that signal data 127, as analyzed as described with respect to Tables 1-5, may ultimately indicate, define, or classify signal data (e.g., EMG signal data of movements of a user over time) as any one or more of "inconsistent," "liftoff," "noisy," "quiet," "indistinct," "similar," "early," "late," "weak," or "strong," etc.

In various embodiments, if user 123 receives an indication of poor calibration (116), a message 118 will be generated to the user 123 based on the analyzed and categorized signal data 127. For example, system 100, based on signal data 127, may provide user 123 with one or more messages (117) related to the signal data 127. Message 118 be a list of one or more possible message classes that would be provided to user 123 after system 100 had determined that the signal data 127 collected was inadequate (116). As illustrated by the embodiment of Figure 1, a message 118 can be one or more of the following messages 118: "inconsistent," "liftoff," "noisy," "quiet," "indistinct," "similar," "early," "late," "weak," "strong," and/or "class-off" (not shown). Each message 118 may be generated based on the analysis of the signal data 127 and provided as feedback (112) to the user 123 through virtual user interface 102. For example, Figure 3 illustrates an example representation of a virtual user interface 102 demonstrating each connected prosthetic device (121), calibration strength for multiple motions (e.g., indicated motions 134, i.e., prompted actions) of system 100 for the coaching of exoprosthetic users, including messages and training tips, in accordance with various embodiments disclosed herein. For example, Figure 3 illustrates a user interface indicator for each connected prosthetic device (121) to system 100, where, for the embodiment of Figure 3, includes each of an "elbow," a "wrist," and a "hand" of a prosthetic device, e.g., prosthetic device 124. EMG signal data for calibration may be measured during time periods or calibration sessions indicated by orange (or other color) circle(s) on virtual user interface 102, where the user is requested to make an indicated motion/prompted action during a time period when the orange circle appears, or otherwise completes its animation, on virtual user interface 102.

The virtual user interface 102 may represent the signal data 127 as a data quality metric 114, where the message 118 may be provided to the user 123 along with a recommended procedure for optimization 133. For example, as shown for Figure 3, message 118 (117) may comprise a "liftoff message" indicating a "significant loss of electrode-skin contact detected." The liftoff message, and additional example messages, corresponding with messages 118 of Figure 1 (and related issues, descriptions, recommendations, and tips), are illustrated below in Table 6.

**TABLE 6 (Calibration Quality Metrics and Messages)**

| Message type (Calibration issue class type) | Description | Message(s) to User | Tip(s) to User |
|---|---|---|---|
| NO_MESSAGE | Implemented when user is provided with no message (115) as shown for Figure 1 | N/A | N/A |
| NOISY_NM | Implemented when there is enough variation in the EMG signal to indicate user muscle contraction or some other constantly changing noise. Such noise causes problems with a no-motion reallocation algorithm (too aggressive, motions hard to access) and often indicates issues with EMG signal quality or the user's understanding of the prompts/messages | "There was quite a bit of noisy signal during recording of Relax/No Motion." | "Make sure all electrodes are making contact. "Be sure not to make muscle contractions when Relax/No Motion is being recorded." |

(continued)

| Message type (Calibration issue class type) | Description | Message(s) to User | Tip(s) to User |
|---|---|---|---|
| QUIET_NM | Implemented when no variation in the EMG signal, as typically expected from electrodes being over muscle and supporting the user's body weight, is detected. Such lack of EMG signals can cause the no-motion reallocation algorithm to be too permissive (missing late/early issues amplified) and may make motions (when the prosthetic device is used) more likely when the user does not intend such motions | "Your Relax/No Motion signals were super quiet."; or "Absent signal(s) during recording of Relax/No Motion." | "To make everything a little more robust, try wiggling your fingers and/or moving your arm around when No Motion is being recorded." |
| INDISTINCT | Implemented when two or more calibration classes are detected/ confused | "Your [X, Y, and Z] are very similar."; or "Your [X, Y, and Z] are somewhat similar."; or<br><br>"Your [X, Y, and Z] are a little bit similar." | For better performance, try calibrating [X, Y, and Z] with a more distinct feel. |
| TOO_SIMILAR | Implemented when a specific pair of calibration classes are likely to be confused. In such cases, a message for either INDISTINCT or TOO _SIMILAR will be indicated based on severity of the EMG signal quality. | "Your [X] is very similar to [Y]."; or "Your [X] is somewhat similar to [Y]."; or "Your [X] is a bit similar to [Y]." | For better performance, try calibrating [X and Y] with a more distinct feel. |
| MISSING_EARLY | Implemented for a late user reaction to a prompted action, such that the beginning EMG signal data is reallocated. | "You started [X] quite late during calibration." | "For the system to work at its best, be sure to hold [X] throughout the whole orange circle during calibration." |
| MISSING_LATE | Implemented when the user relaxes too early from the indicated prompted action. | "You stopped holding [X] quite early during calibration."; or "You stopped holding [X] a little bit early during calibration." | "For the system to work at its best, be sure to hold [X] throughout the whole orange circle during calibration." |

(continued)

| Message type (Calibration issue class type) | Description | Message(s) to User | Tip(s) to User |
|---|---|---|---|
| INCONSISTENT | Tracks reallocation, but triggers for large amounts of reallocation, regardless of whether the calibration is at the beginning, middle, or end of a prompted action. Differs from TOO_WEAK in that data is inconsistently missing (sparse EMG signal), not just quiet (low EMG signal). | "Your [X] was cutting in and out a lot during calibration."; or "Your [X] was cutting in and out during calibration."; or "Your [X] was cutting in and out somewhat during calibration."; or "Your [X] was cutting in and out a little bit during calibration." | "For the system to work at its best, be sure to hold [X] throughout the whole orange circle during calibration." |
| TOO_WEAK | Detects when the EMG signal for a contraction is not much higher than no | "Your [X] was too soft during calibration."; or | "Make sure the system gets your data; try calibrating |
| | motion (no or low EMG signal). TOO_WEAK also tracks from reallocation, such that TOO_WEAK competes directly with INCONSISTENT but differs in that EMG signal data is detected (i.e., a low EMG signal data is detected). | "Your [X] was a little too soft during calibration."; or "Your [X] was somewhat soft during calibration."; or "Your [X] was soft during calibration." | [X] a little bit stronger." |
| TOO_STRONG | Implemented when the EMG signal for a contraction is from a user who is likely straining/exerting themselves (causing a strong EMG signal)? | "Your [X] was too hard during calibration."; or "Your [X] was a little too hard during calibration." | "To help the system perform better and to keep you from getting muscle fatigue, try calibrating [X] a little bit softer." |
| LIFTOFF | Provided as a global "warning" message in the event that significant liftoff of electrode(s) from the user's skin was detected during prosthetic device calibration. | Some electrodes are not making good skin contract during calibration for [X]. | You are not likely to get good performance until electrode-skin contact issues are addressed. |

In the above Table 6, different indicated motions/prompted actions (e.g., elbow motion ("flex" and/or "extend"), wrist motion ("pronate" and/or "supinate"), and/or hand motion ("open," "close," "tool," "key," and/or "pinch") as illustrated by Figure 3), are provided to, or otherwise represented by, "[X]," "[Y]," and/or "[Z]," where the various indicated motions/prompted actions may be filled into the messages (via the "[X]," "[Y]," and/or "[Z]" as placeholder locations) and presented to the user. In addition, each of the message types (calibration issue class types) as shown above in Table 6 may be associated with a data quality metric 114 penalty, where a data quality metric 114 may be decreased or otherwise altered or updated when a message types (calibration issue class types) is detected. For example, detection of a TOO_WEAK message type (calibration issue class type) may cause the loss of up to 4 data quality metrics 114, e.g., which may cause a corresponding loss of up to 4 star ratings as displayed on user interface (e.g., 125 or 126) as described herein. As another example, detection of a MISSING_LATE message type (calibration issue class type) may cause the loss of up to 2 data quality metrics 114, e.g., which may cause a corresponding loss of up to 2 star ratings as displayed on user interface (e.g., 125 or 126) as described herein. It is to be understood that the alteration of the data quality metrics 114/star ratings may be adjusted, or otherwise set, based on the severity, or otherwise impact, that a user's insufficient calibration session, as indicated by a corresponding message type (calibration issue class type), causes to the collection of EMG data for calibration and/or the resulting calibration of the prosthetic device, e.g., prosthetic device 124 itself.

Message 118 may further include a recommended procedure for optimization 133 of signal data 127. A given recommended procedure for optimization 133 corresponds to each connected prosthetic device (121) connected to the system and its related indicated motion 134. For example, as shown for Figure 3, the recommended procedure of optimization is to calibrate the "Hand" prosthetic device (121) (e.g., of prosthetic device 124) and its related "open" indicated motion 134, because the "open" motion of the "hand" device has a lowest signal data 127 quality or quality metric 114 (e.g., a quality metric of three stars). Still further, as shown for Figure 3, recommended procedure for optimization 133 may be, or cause to be generated, a tip based on the message class. For example, as shown in Figure 3, the message class is "liftoff," where the tip to user 123 is generated and/or displayed as: "good control performance should not be expected until electrode-skin contact issues are addressed ...."

As shown for Figure 1, system 100 then may end (108) the calibration protocol 109. The recommended procedure for optimization 133 may be recorded in a system memory 135 (e.g., a system memory of, or as communicatively coupled to a computing or electronic device, such as prosthetic device 124, computer 125a, mobile device 126a, connection module 132, myoelectric prosthetic controller, or otherwise of system 100) so user 123 can access the recommended procedure for optimization 133 at a later date, and review their collected signal data 127 in relation to a given prosthetic or indicated motion 134 as shown and described herein and/or as illustrated by any of the Figures herein.

ASPECTS OF THE DISCLOSRE

1. A system for the coaching of exoprosthetic users comprising: an apparatus for the collection of signal data; a button; and a software component.

2. A system for the input of signal data comprising: a plurality of electrodes.

3. A button comprising: a housing; an indicator; and a tactile interface.

4. A software component comprising: a user interface; and a pattern recognition component.

5. The system for the input of signal data of Aspect 2, wherein the plurality of electrodes comprise a composition of an electrically conductive material.

6. The system for the input of signal data of Aspect 2, further comprising: a method for the communicating of the signal data to the software component of Aspect 4.

7. The button of Aspect 3, wherein the indicator is comprised of: an apparatus for visual stimulus; an apparatus for auditory stimulus; and/or an apparatus for tactile stimulus.

8. The software component of Aspect 4, wherein the user interface further comprises: a system for the feedback of information to the user.

9. The User Interface of Aspect 8, wherein the system for the feedback of information to the user is further comprised of: a quality metric, identifying an objective level of calibration quality; and/or a message, identifying probable causes of poor signal data.

10. The system for the feedback of information to the user of Aspect 9, wherein the message is further comprised of: an indication of the cause for non-optimal signal data input; and a recommended procedure for optimizing the signal data input.

11. The software component of Aspect 4, wherein the user interface is further comprised of: a virtual application that can be interacted with by the user.

12. The software component of Aspect 4, wherein the user interface is further comprised of: a calibration procedure; and a set of instructions to guide the user through the calibration procedure.

13. The software component of Aspect 4, wherein the user interface is further comprised of: a selection of a prostheses connection; a selection of one or more movements; an indication of signal data input; or, an indication of signal data output.

14. The User Interface of Aspect 13, wherein the indication of signal data input is further comprised of: an identification of connected hardware; and an identification of the status of connected hardware.

15. The software component of Aspect 4, wherein the user interface is further comprised of: a system for the user to directly monitor the signal data, in real time.

16. The software component of Aspect 4, wherein the pattern recognition component further comprises: a system for the receiving of signal data; a system for the analysis of signal data; and a system for the output of signal data.

17. The software component of Aspect 4, wherein the pattern recognition component further comprises: an adaptive machine learning system to recognize the users unique signal data.

18. The pattern recognition component of Aspect 17, wherein the adaptive machine learning system further comprises: a system for the recognition of a user's unique signal data in reference to a particular motion by the user.

19. The software component of Aspect 4, wherein the pattern recognition component further comprises: a system for identifying and categorizing signal data from a user.

20. The software component of Aspect 4, wherein the pattern recognition component further comprises: a system for the communication of the categorized signal data to the user.

21. The user interface of Aspect 11, wherein the virtual application further comprises: an installation method involving downloaded content from the internet; an installation method involving uploadable content from a physical disk or drive; and the installation methods having compatibility with digital operating systems.

22. The button of Aspect 3, wherein the tactile interface further comprises: a system to initiate the calibration procedure of Aspect 12 from the prostheses.

23. The user interface of Aspect 11, wherein the virtual application further comprises: a system to initiate the calibration procedure of Aspect 12 from the virtual application.

The foregoing aspects of the disclosure are exemplary only and not intended to limit the scope of the disclosure.

ADDITIONAL ASPECTS OF THE DISCLOSRE

1. An electromyographic control system configured to coach prosthetic users to calibrate prosthetic devices, the electromyographic control system comprising: a myoelectric prosthetic controller configured to control a prosthetic device; an electromyographic software component communicatively coupled to a plurality of electrodes in myoelectric contact with a user, wherein the electromyograph software component is configured to perform an analysis of electromyographic (EMG) signal data of the user, the EMG signal data received from the plurality of electrodes; and a user interface configured to provide, based on the analysis of the EMG signal data, a feedback indication to the user as to a calibration quality of the EMG signal data, wherein the user interface is configured to initiate a calibration procedure to calibrate the myoelectric prosthetic controller, and wherein the user interface comprises at least one of: (i) a button user interface including a calibration button, or (ii) a virtual user interface configured to display the feedback indication as at least one of: (a) a quality metric corresponding to the calibration quality of the EMG signal data, or (b) a message corresponding to the calibration quality of the EMG signal data.

2. The electromyographic control system of additional aspect 1, wherein the message comprises at least one of (a) an indication of a cause for a non-optimal signal data input of the EMG signal data, or (b) a recommended procedure for optimizing signal data input.

3. The electromyographic control system of any of additional aspects 1 or 2, wherein the calibration procedure is initiated during a calibration session, wherein the virtual user interface provides a recommended procedure for optimizing signal data input, and wherein a further calibration procedure is initiated from the user interface during a further calibration session to recalibrate the myoelectric prosthetic controller based on the recommended procedure.

4. The electromyographic control system of additional aspect 3, wherein the further calibration session is configured to facilitate at least one of (a) deleting EMG signal data corresponding to one or more data sets or movements, (b) adding EMG signal data corresponding to one or more data sets or movements, (c) replacing EMG signal data corresponding to one or more data sets or movements with new EMG signal data.

5. The electromyographic control system of any of the previous additional aspects, wherein the myoelectric prosthetic controller is calibrated to control the prosthetic device based on the EMG signal data.

6. The electromyographic control system of any of the previous additional aspects, wherein the calibration button is configured to provide the feedback indication by at least one of an auditory stimulus, a tactile stimulus, or a visual stimulus.

7. The electromyographic control system of any of the previous additional aspects, wherein virtual user interface displays a visualization of the EMG signal data in real time.

8. The electromyographic control system of any of the previous additional aspects, wherein the calibration procedure comprises the virtual user interface instructing the user to perform one or more indicated motions in relation to the prosthetic device, and wherein the one or more indicated motions produce the EMG signal data as received from the plurality of electrodes.

9. The electromyographic control system of additional aspect 8, wherein the virtual user interface is configured to receive one or more selections indicating at least one of the one or more indicated motions for the user to perform.

10. The electromyographic control system of any of the previous additional aspects, wherein the electromyographic software component further comprises a pattern recognition component configured to analyze the EMG signal data of the user, the pattern recognition component further configured to identify or categorize the EMG signal data of the user based on a particular motion performed by the user.

11. The electromyographic control system of additional aspect 10, wherein the pattern recognition component comprises an adaptive machine learning component configured to determine the particular motion performed by the user based on the EMG signal data of the user.

12. The electromyographic control system of additional aspect 11, wherein the adaptive machine learning component is further configured to determine an appropriate feedback indication based on the EMG signal data of the user.

13. The electromyographic control system of any of the previous additional aspects, wherein the user interface is configured to reset calibration data of the user to calibrate the myoelectric prosthetic controller.

14. An electromyographic control method for coaching prosthetic users to calibrate prosthetic devices, the electromyographic control method comprising: receiving, by an electromyographic software component communicatively coupled to a plurality of electrodes in myoelectric contact with a user, electromyographic (EMG) signal data from the plurality of electrodes; analyzing, by the electromyograph software component, the EMG signal data of the user; providing to a user interface, based on analyzing the EMG signal data, a feedback indication to the user as to a calibration quality of the EMG signal data; and initiating, based on the calibration quality of the EMG signal data, a calibration procedure to calibrate a myoelectric prosthetic controller, the myoelectric prosthetic controller configured to control a prosthetic device, wherein the user interface comprises at least one of: (i) a button user interface including a calibration button, or (ii) a virtual user interface configured to display the feedback indication as at least one of: (a) a quality metric corresponding to the calibration quality of the EMG signal data, or (b) a message corresponding to the calibration quality of the EMG signal data.

15. The electromyographic control method of additional aspect 14, wherein the calibration procedure is initiated during a calibration session, wherein the virtual user interface provides a recommended procedure for optimizing signal data input, and wherein a further calibration procedure is initiated from the user interface during a further calibration session to recalibrate the myoelectric prosthetic controller based on the recommended procedure.

16. The electromyographic control method of additional aspects 14 or 15, wherein the calibration procedure comprises the virtual user interface instructing the user to perform one or more indicated motions in relation to the prosthetic device, and wherein the one or more indicated motions produce the EMG signal data as received from the plurality of electrodes.

17. The electromyographic control method of any of additional aspects 14 to 17, wherein the electromyographic software component further comprises a pattern recognition component configured to analyze the EMG signal data of the user, the pattern recognition component further configured to identify or categorize the EMG signal data of the user based on a particular motion performed by the user.

18. The electromyographic control method of additional aspect 17, wherein the pattern recognition component comprises an adaptive machine learning component configured to determine the particular motion performed by the user based on the EMG signal data of the user.

19. A tangible, non-transitory computer-readable medium storing instructions for coaching prosthetic users to calibrate prosthetic devices, that when executed by one or more processors cause the one or more processors to: receive, by an electromyographic software component communicatively coupled to a plurality of electrodes in myoelectric contact with a user, electromyographic (EMG) data from the plurality of electrodes; analyze, by the electromyograph software component, the EMG signal data of the user; provide to a user interface, based on analyzing the EMG signal data, a feedback indication to the user as to a calibration quality of the EMG signal data; and initiate, based on the calibration quality of the EMG signal data, a calibration procedure to calibrate a myoelectric prosthetic controller, the myoelectric prosthetic controller configured to control a prosthetic device, wherein the user interface comprises at least one of: (i) a button user interface including a calibration button, or (ii) a virtual user interface configured to display the feedback indication as at least one of: (a) a quality metric corresponding to the calibration quality of the EMG signal data, or (b) a message corresponding to the calibration quality of the EMG signal data.

20. The tangible, non-transitory computer-readable medium of additional aspect 19, wherein the calibration procedure is initiated during a calibration session, wherein the virtual user interface provides a recommended procedure for optimizing signal data input, and wherein a further calibration procedure is initiated from the user interface during a further calibration session to recalibrate the myoelectric prosthetic controller based on the recommended procedure.

The foregoing additional aspects of the disclosure are exemplary only and not intended to limit the scope of the disclosure.

ADDITIONAL CONSIDERATIONS

Although the disclosure herein sets forth a detailed description of numerous different embodiments, it should be understood that the legal scope of the description is defined by the words of the claims set forth at the end of this patent and equivalents. The detailed description is to be construed as exemplary only and does not describe every possible embodiment since describing every possible embodiment would be impractical. Numerous alternative embodiments may be implemented, using either current technology or technology developed after the filing date of this patent, which would still fall within the scope of the claims.

The following additional considerations apply to the foregoing discussion. Throughout this specification, plural instances may implement components, operations, or structures described as a single instance. Although individual operations of one or more methods are illustrated and described as separate operations, one or more of the individual operations may be performed concurrently, and nothing requires that the operations be performed in the order illustrated. Structures and functionality presented as separate components in example configurations may be implemented as a combined structure or component. Similarly, structures and functionality presented as a single component may be implemented as separate components. These and other variations, modifications, additions, and improvements fall within the scope of the subject matter herein.

Additionally, certain embodiments are described herein as including logic or a number of routines, subroutines, applications, or instructions. These may constitute either software (e.g., code embodied on a machine-readable medium or in a transmission signal) or hardware. In hardware, the routines, etc., are tangible units capable of performing certain operations and may be configured or arranged in a certain manner. In example embodiments, one or more computer systems (e.g., a standalone, client or server computer system) or one or more hardware modules of a computer system (e.g., a processor or a group of processors) may be configured by software (e.g., an application or application portion) as a hardware module that operates to perform certain operations as described herein.

In various embodiments, a hardware module may be implemented mechanically or electronically. For example, a hardware module may comprise dedicated circuitry or logic that is permanently configured (e.g., as a special-purpose processor, such as a field programmable gate array (FPGA) or an application-specific integrated circuit (ASIC)) to perform certain operations. A hardware module may also comprise programmable logic or circuitry (e.g., as encompassed within a general-purpose processor or other programmable processor) that is temporarily configured by software to perform certain operations. It will be appreciated that the decision to implement a hardware module mechanically, in dedicated and permanently configured circuitry, or in temporarily configured circuitry (e.g., configured by software) may be driven by cost and time considerations.

Accordingly, the term "hardware module" or "hardware component" should be understood to encompass a tangible entity, be that an entity that is physically constructed, permanently configured (e.g., hardwired), or temporarily configured (e.g., programmed) to operate in a certain manner or to perform certain operations described herein. Considering embodiments in which hardware modules are temporarily configured (e.g., programmed), each of the hardware modules need not be configured or instantiated at any one instance in time. For example, where the hardware modules comprise a general-purpose processor configured using software, the general-purpose processor may be configured as respective different hardware modules at different times. Software may accordingly configure a processor, for example, to constitute a particular hardware module at one instance of time and to constitute a different hardware module at a different instance of time.

Hardware modules may provide information to, and receive information from, other hardware modules. Accordingly, the described hardware modules may be regarded as being communicatively coupled. Where multiple of such hardware modules exist contemporaneously, communications may be achieved through signal transmission (e.g., over appropriate circuits and buses) that connect the hardware modules. In embodiments in which multiple hardware modules are configured or instantiated at different times, communications between such hardware modules may be achieved, for example, through the storage and retrieval of information in memory structures to which the multiple hardware modules have access. For example, one hardware module may perform an operation and store the output of that operation in a memory device to which it is communicatively coupled. A further hardware module may then, at a later time, access the memory device to retrieve and process the stored output. Hardware modules may also initiate communications with input or output devices, and may operate on a resource (e.g., a collection of information).

The various operations of example methods described herein may be performed, at least partially, by one or more processors that are temporarily configured (e.g., by software) or permanently configured to perform the relevant operations. Whether temporarily or permanently configured, such processors may constitute processor-implemented modules that operate to perform one or more operations or functions. The modules referred to herein may, in some example embodiments, comprise processor-implemented modules.

Similarly, the methods or routines described herein may be at least partially processor-implemented. For example, at least some of the operations of a method may be performed by one or more processors or processor-implemented hardware modules. The performance of certain of the operations may be distributed among the one or more processors, not only residing within a single machine, but deployed across a number of machines. In some example embodiments, the processor or processors may be located in a single location, while in other embodiments the processors may be distributed across a number of locations.

The performance of certain of the operations may be distributed among the one or more processors, not only residing within a single machine, but deployed across a number of machines. In some example embodiments, the one or more processors or processor-implemented modules may be located in a single geographic location (e.g., within a home environment, an office environment, or a server farm). In other embodiments, the one or more processors or processor-implemented modules may be distributed across a number of geographic locations.

This detailed description is to be construed as exemplary only and does not describe every possible embodiment, as describing every possible embodiment would be impractical, if not impossible. A person of ordinary skill in the art may implement numerous alternate embodiments, using either current technology or technology developed after the filing date of this application.

Those of ordinary skill in the art will recognize that a wide variety of modifications, alterations, and combinations can be made with respect to the above described embodiments without departing from the scope of the invention, and that such modifications, alterations, and combinations are to be viewed as being within the ambit of the inventive concept.

The patent claims at the end of this patent application are not intended to be construed under 35 U.S.C. § 112(f) unless traditional means-plus-function language is expressly recited, such as "means for" or "step for" language being explicitly recited in the claim(s). The systems and methods described herein are directed to an improvement to computer functionality, and improve the functioning of conventional computers.

The present teaching may also extend to the features of one or more of the following numbered clauses:

Clauses:

1. An electromyographic control system configured to coach prosthetic users to calibrate prosthetic devices, the electromyographic control system comprising:

a myoelectric prosthetic controller configured to control a prosthetic device;
an electromyographic software component communicatively coupled to a plurality of electrodes in myoelectric contact with a user, wherein the electromyograph software component is configured to perform an analysis of electromyographic (EMG) signal data of the user, the EMG signal data received from the plurality of electrodes; and
a user interface configured to provide, based on the analysis of the EMG signal data, a feedback indication to the user as to a calibration quality of the EMG signal data,
wherein the user interface is configured to initiate a calibration procedure to calibrate the myoelectric prosthetic controller, and
wherein the user interface comprises at least one of:

(i) a button user interface including a calibration button, or
(ii) a virtual user interface configured to display the feedback indication as at least one of: (a) a quality metric corresponding to the calibration quality of the EMG signal data, or (b) a message corresponding to the calibration quality of the EMG signal data.

2. The electromyographic control system of clause 1, wherein the message comprises at least one of (a) an indication of a cause for a non-optimal signal data input of the EMG signal data, or (b) a recommended procedure for optimizing signal data input.

3. The electromyographic control system of clause 1,

wherein the calibration procedure is initiated during a calibration session,
wherein the virtual user interface provides a recommended procedure for optimizing signal data input, and
wherein a further calibration procedure is initiated from the user interface during a further calibration session to recalibrate the myoelectric prosthetic controller based on the recommended procedure.

4. The electromyographic control system of clause 3, wherein the further calibration session is configured to facilitate at least one of (a) deleting EMG signal data corresponding to one or more data sets or movements, (b) adding EMG signal data corresponding to one or more data sets or movements, (c) replacing EMG signal data corresponding to one or more data sets or movements with new EMG signal data.

5. The electromyographic control system of clause 1, wherein the myoelectric prosthetic controller is calibrated to control the prosthetic device based on the EMG signal data.

6. The electromyographic control system of clause 1, wherein the calibration button is configured to provide the feedback indication by at least one of an auditory stimulus, a tactile stimulus, or a visual stimulus.

7. The electromyographic control system of clause 1, wherein virtual user interface displays a visualization of the EMG signal data in real time.

8. The electromyographic control system of clause 1,

wherein the calibration procedure comprises the virtual user interface instructing the user to perform one or more indicated motions in relation to the prosthetic device, and
wherein the one or more indicated motions produce the EMG signal data as received from the plurality of electrodes.

9. The electromyographic control system of clause 8, wherein the virtual user interface is configured to receive one or more selections indicating at least one of the one or more indicated motions for the user to perform.

10. The electromyographic control system of clause 1, wherein the electromyographic software component further comprises a pattern recognition component configured to analyze the EMG signal data of the user, the pattern recognition component further configured to identify or categorize the EMG signal data of the user based on a particular motion performed by the user.

11. The electromyographic control system of clause 10, wherein the pattern recognition component comprises an adaptive machine learning component configured to determine the particular motion performed by the user based on the EMG signal data of the user.

12. The electromyographic control system of clause 11, wherein the adaptive machine learning component is further configured to determine an appropriate feedback indication based on the EMG signal data of the user.

13. The electromyographic control system of clause 1, wherein the user interface is configured to reset calibration data of the user to calibrate the myoelectric prosthetic controller.

14. An electromyographic control method for coaching prosthetic users to calibrate prosthetic devices, the electromyographic control method comprising:

receiving, by an electromyographic software component communicatively coupled to a plurality of electrodes in myoelectric contact with a user, electromyographic (EMG) signal data from the plurality of electrodes;
analyzing, by the electromyograph software component, the EMG signal data of the user;
providing to a user interface, based on analyzing the EMG signal data, a feedback indication to the user as to a calibration quality of the EMG signal data; and
initiating, based on the calibration quality of the EMG signal data, a calibration procedure to calibrate a myoelectric prosthetic controller, the myoelectric prosthetic controller configured to control a prosthetic device,
wherein the user interface comprises at least one of:

(i) a button user interface including a calibration button, or
(ii) a virtual user interface configured to display the feedback indication as at least one of: (a) a quality metric corresponding to the calibration quality of the EMG signal data, or (b) a message corresponding to the calibration quality of the EMG signal data.

15. The electromyographic control method of clause 14,

wherein the calibration procedure is initiated during a calibration session,
wherein the virtual user interface provides a recommended procedure for optimizing signal data input, and
wherein a further calibration procedure is initiated from the user interface during a further calibration session to recalibrate the myoelectric prosthetic controller based on the recommended procedure.

16. The electromyographic control method of clause 14,

wherein the calibration procedure comprises the virtual user interface instructing the user to perform one or more indicated motions in relation to the prosthetic device, and
wherein the one or more indicated motions produce the EMG signal data as received from the plurality of electrodes.

17. The electromyographic control method of clause 14, wherein the electromyographic software component further comprises a pattern recognition component configured to analyze the EMG signal data of the user, the pattern recognition component further configured to identify or categorize the EMG signal data of the user based on a particular motion performed by the user.

18. The electromyographic control method of clause 17, wherein the pattern recognition component comprises an adaptive machine learning component configured to determine the particular motion performed by the user based on the EMG signal data of the user.

19. A tangible, non-transitory computer-readable medium storing instructions for coaching prosthetic users to calibrate prosthetic devices, that when executed by one or more processors cause the one or more processors to:

receive, by an electromyographic software component communicatively coupled to a plurality of electrodes in myoelectric contact with a user, electromyographic (EMG) data from the plurality of electrodes;
analyze, by the electromyograph software component, the EMG signal data of the user;
provide to a user interface, based on analyzing the EMG signal data, a feedback indication to the user as to a calibration quality of the EMG signal data; and

initiate, based on the calibration quality of the EMG signal data, a calibration procedure to calibrate a myoelectric prosthetic controller, the myoelectric prosthetic controller configured to control a prosthetic device, wherein the user interface comprises at least one of:

(i) a button user interface including a calibration button, or
(ii) a virtual user interface configured to display the feedback indication as at least one of: (a) a quality metric corresponding to the calibration quality of the EMG signal data, or (b) a message corresponding to the calibration quality of the EMG signal data.

20. The tangible, non-transitory computer-readable medium of clause 19,

wherein the calibration procedure is initiated during a calibration session,
wherein the virtual user interface provides a recommended procedure for optimizing signal data input, and
wherein a further calibration procedure is initiated from the user interface during a further calibration session to recalibrate the myoelectric prosthetic controller based on the recommended procedure.

## Claims

1. An electromyographic control system (100) configured to coach prosthetic users to calibrate prosthetic devices, the electromyographic control system (100) comprising:

   a myoelectric prosthetic controller configured to control a prosthetic device (124);
   an electromyographic software component (136) communicatively coupled to a plurality of electrodes (122) in myoelectric contact with a user (123), wherein the electromyograph software component (136) is configured to perform an analysis of electromyographic, EMG, signal data (127) of the user (123), the EMG signal data (127) received from the plurality of electrodes (122); and
   **characterized in that** the system further comprises:

   a user interface configured to provide, based on the analysis of the EMG signal data (127), a feedback indication to the user (123) as to a calibration quality of the EMG signal data (127), wherein the user interface is configured to initiate a calibration procedure to calibrate the myoelectric prosthetic controller, wherein the user interface comprises a virtual user interface (102) configured to display the feedback indication as at least one of: (a) a quality metric (114) corresponding to the calibration quality of the EMG signal data (127), or (b) a message (118) corresponding to the calibration quality of the EMG signal data (127), and
   wherein the software component renders the user interface and is coupled to the prosthetic device.

2. The electromyographic control system (100) of claim 1, wherein the user interface comprises the virtual user interface (102), and wherein the message (118) comprises at least one of (a) an indication of a cause for a non-optimal signal data input of the EMG signal data (127), or (b) a recommended procedure for optimizing signal data input.

3. The electromyographic control system (100) of claim 1,

   wherein the user interface initiates the calibration procedure during a calibration session,
   wherein the virtual user interface (102) provides a recommended procedure for optimizing signal data input, and
   wherein the user interface initiates a further calibration procedure during a further calibration session to recalibrate the myoelectric prosthetic controller based on the recommended procedure.

4. The electromyographic control system (100) of claim 3, wherein the further calibration session causes the electromyographic control system to facilitate at least one of (a) deleting EMG signal data (127) corresponding to one or more data sets or movements, (b) adding EMG signal data (127) corresponding to one or more data sets or movements, (c) replacing EMG signal data (127) corresponding to one or more data sets or movements with new EMG signal data.

5. The electromyographic control system (100) of claim 1, wherein the myoelectric prosthetic controller is calibrated to control the prosthetic device (124) based on the EMG signal data (127).

6. The electromyographic control system (100) of claim 1, wherein the calibration button is configured to provide the feedback indication by at least one of an auditory stimulus, a tactile stimulus, or a visual stimulus.

7. The electromyographic control system (100) of claim 1, wherein virtual user interface (102) displays a visualization of the EMG signal data (127) in real time.

8. The electromyographic control system (100) of claim 1,

   wherein the calibration procedure comprises the virtual user interface (102) instructing the user (123) to perform one or more indicated motions in relation to the prosthetic device (124), and
   wherein the one or more indicated motions produce the EMG signal data (127) as received from the plurality of electrodes (122).

9. The electromyographic control system (100) of claim 8, wherein the virtual user interface (102) is configured to receive one or more selections indicating at least one of the one or more indicated motions for the user (123) to perform.

10. The electromyographic control system (100) of claim 1, wherein the electromyographic software component (136) further comprises a pattern recognition component configured to analyze the EMG signal data (127) of the user (123), the pattern recognition component further configured to identify or categorize the EMG signal data (127) of the user (123) based on a particular motion performed by the user (123).

11. The electromyographic control system (100) of claim 10, wherein the pattern recognition component comprises an adaptive machine learning component configured to determine the particular motion performed by the user (123) based on the EMG signal data (127) of the user (123).

12. The electromyographic control system (100) of claim 11, wherein the adaptive machine learning component is further configured to determine an appropriate feedback indication based on the EMG signal data (127) of the user (123).

13. The electromyographic control system (100) of claim 1, wherein the user interface is configured to reset calibration data (104) of the user (123) to calibrate the myoelectric prosthetic controller.

14. An electromyographic control method for coaching prosthetic users to calibrate prosthetic devices, the electromyographic control method implemented by the electromyographic control system (100) in any of the preceding claims.

15. A tangible, non-transitory computer-readable medium storing instructions for coaching prosthetic users to calibrate prosthetic devices as implemented by the electromyographic control system (100) in any of the preceding claims.

FIG. 1

119  120  102

CALIBRATE

**FAIR** — 114

★★★☆☆ ⓘ — 114

NO SIGNAL CONTACT

Virtual Arm — 107
Prosthetic Device

136  135  125
(Computer-
based UI)

125a (Computer)

**FIG. 2**

**FIG. 3**

EP 4 684 758 A2

FIG. 4

FIG. 4B

EP 4 684 758 A2

**117,118** EMG signal

Noisy

127

Time (t)

**117,118** EMG signal

Quiet

127

Time (t)

**117,118** EMG signal

Inconsistent

127

Time (t)

**FIG. 5A**

**FIG. 5B**

**FIG. 6A**

**FIG. 6B**

**REFERENCES CITED IN THE DESCRIPTION**

**Patent documents cited in the description**

• US 62807306